# EUROPÄISCHE PATENTANMELDUNG

(11) **EP 0 663 204 A1**
(43) Veröffentlichungstag der Anmeldung: **19.07.1995**
(21) Anmeldenummer: 94119390.6
(22) Anmeldetag: 08.12.1994
(51) Int. Cl.: A61K 7/13

(54) **Haarfärbemittel**

(30) Priorität: 13.01.1994 DE 4400756
(71) Anmelder: GOLDWELL GmbH, D-64297 Darmstadt (DE)
(72) Erfinder: Lorenz, Heribert, D-64401 Gross-Bieberau (DE); Tennigkeit, Jürgen, Dr., D-64342 Seeheim (DE); Kufner, Frank, D-64297 Darmstadt (DE)

(57) **Zusammenfassung**

Ein toxikologisch und dermatologisch unbedenkliches Haarfärbemittel enthält als Entwicklersubstanz 1-Amino-4-bis-(β-hydroxyethyl)-aminobenzol und zusätzlich mindestens eine der Komponenten 4,6-Diaminopyrimidin, 4-Amino-2,6-dihydroxypyrimidin, 4,5,6-Triaminopyrimidin, 2,4-Diamino-6-hydroxypyrimidin, 2,4,5-Trihydroxypyrimidin, 2,4,6-Trihydroxypyrimidin, 2-Amino-4-methylpyrimidin, 2-Amino-4-hydroxy-6-methylpyrimidin, 4,6-Dihydroxy-2-methylpyrimidin, 3,4-Diamino-5-hydroxypyrazol und/oder 3-Amino-5,6-dimethyl-1,2,4-triazin bzw. deren wasserlösliche Salze. Mit dieser Zusammensetzung wurden Haarfärbungen mit exzellenter Licht-, Wasch- und Dauerwellbeständigkeit erzielt.

## Beschreibung

Die vorliegende Erfindung betrifft ein neues Haarfärbemittel für menschliches Haar, das insbesondere verbesserte Farbeigenschaften und eine ausgezeichnete toxikologische und dermatologische Verträglichkeit aufweist.

Haarfärbemittel enthalten, wie in Fachkreisen allgemein bekannt, mindestens ein Oxidationsfarbstoff-Vorprodukt, eine sogenannte Entwicklerkomponente, und mindestens einen Reaktionspartner (Kuppler), die unmittelbar vor der Haarfärbung mit Wasserstoffperoxid zusammengebracht werden und dann auf dem Haar die gewünschte Farbreaktion ergeben. Zur Feineinstellung des gewünschten Farbtones werden einer solchen Mischung üblicherweise auch Nuanceure zugesetzt.

Übliche Entwicklersubstanzen sind insbesondere aromatische Diamine wie 1,4-Diaminobenzol (p-Phenylendiamin) und 1-Methyl-2,5-diaminobenzol (p-Toluylendiamin). Diese Substanzen sind hinsichtlich ihrer dermatologischen Eigenschaften für manche Benutzer nicht optimal. Es bestand daher ein Bedürfnis, weitere Entwicklersubstanzen mit verbesserten dermatologischen und toxikologischen Eigenschaften zu finden.

Eine solche weitere bekannte Entwicklersubstanz ist 1-Amino-4-bis-(β-hydroxyethyl)-aminobenzol, die als Base oder wasserlösliches Salz, z. B. als Sulfat, zum Einsatz gelangt.

In Kombination mit den üblichen Kupplern wurden bisher jedoch keine herausragenden Farbergebnisse erzielt, weshalb diese Entwicklersübstanz auch nur in sehr geringem Umfang Verwendung gefunden hat.

Die vorliegende Erfindung geht daher von der Aufgabenstellung aus, eine toxikologisch und dermatologisch gut verträgliche Haarfärbezusammensetzung zu entwickeln, die in Kombination mit 1-Amino-4-bis-(β-hydroxyethyl)-aminobenzol-Entwickler eine möglichst lang anhaltende und ausdrucksvolle Haarfärbung ergibt.

Die Lösung dieser Aufgabe besteht erfindungsgemäß darin, 1-Amino-4-bis-(β-hydroxyethyl)-aminobenzol bzw. dessen wasserlösliche Salze als Entwickler enthaltenden Haarfärbemitteln zusätzlich mindestens eine der Komponenten 4,6-Diaminopyrimidin, 4-Amino-2,6-dihydroxpyrimidin, 4,5,6-Triaminopyrimidin, 2,4-Diamino-6-hydroxpyrimidin, 2,4,5-Trihydroxypyrimidin, 2,4,6-Trihydroxpyrimidin, 2-Amino-4-methylpyrimidin, 2-Amino-4-hydroxy-6-methylpyrimidin, 4,6-Dihydroxy-2-methylpyrimidin, 3,4-Diamino-5-hydroxypyrazol und (oder) 3-Amino-5,6-dimethyl-1,2,4-triazin bzw. deren wasserlösliche Salze allein oder im Gemisch mit anderen Kupplern zuzusetzen.

Durch die Kombination dieser speziellen Entwicklersübstanz mit den speziellen Reaktionspartnern werden überraschenderweise, nach Zusatz von Wasserstoffperoxid oder anderen Peroxiden, Haarfärbungen mit exzellenter Lichtechtheit und langer Haltbarkeit, d. h. verbesserter Licht-, Wasch- und Dauerwellbeständigkeit, erreicht.

Darüber hinaus läßt sich mit speziellen Mischungen auch ein ausdrucksvoller blauer Farbton erreichen.

Neben 1-Amino-4-bis-(β-hydroxyethyl)-aminobenzol können, in untergeordneten Mengen, noch weitere Entwicklersubstanzen mitverwendet werden. Bevorzugt sind dabei die aus der EP-A 467 026 bekannten 2,4,5-Triamino-6-hydroxypyrimidin oder 4,5,6-Triamino-2-hydroxypyrimidin. Diese Verbindungen werden, ebenso wie das 1-Amino-4-bis-(β-hydroxyethyl)-aminobenzol, vorzugsweise in Form ihrer wasserlöslichen Salze eingesetzt, sie können jedoch selbstverständlich auch als freie Basen verwendet werden.

Der Anteil an Entwicklersubstanzen in den erfindungsgemäßen Haarfärbemitteln beträgt zwischen etwa 0,05 und 5, vorzugsweise 0,1 und 4, insbesondere 0,5 und 3 Gew.-% der Gesamtzusammensetzung (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haarfärbemitteln etwa im gleichen Anteil wie die Entwicklersubstanzen vor, d. h. also in Mengen von 0,05 bis 5,0, vorzugsweise 0,1 bis 4, insbesondere 0,5 bis 3 Gew.-% der Gesamtzusammensetzung (ohne Oxidationsmittel), wobei sich die Angaben jeweils auf den Anteil an freier Base beziehen.

Auch hier ist es möglich und zuweilen auch zweckmäßig, weitere bekannte Kupplersubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten. Solche Nuanceure sind beispielsweise Nitrofarbstoffe wie 2-Amino-4,6-dinitrophenol, 2-Amino-4-nitrophenol, 2-Amino-6-chlor-4-nitrophenol etc., vorzugsweise in Mengen von etwa 0,05 bis 2,5, insbesondere 0,1 bis 1 Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K.Schrader, "Grundlagen und Rezepturen der Kosmetika", 2. Auflage (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem pH-Bereich von 5,5 bis 6,9, im neutralen als auch im alkalischen Bereich, d. h. zwischen pH 7,1 und 9,5, liegen.

Eine besonders schonende Haarfärbung wird bei Applikation einer schwach sauren Färbemittelzusammensetzung erhalten.

Im folgenden werden verschiedene Ausführungsbeispiele zur Erläuterung der Erfindung gegeben.

Es wurden Farbstoffvorprodukt-Lösungen hergestellt, wobei jeweils 0,068 mol/kg Entwicklersubstanz (1-Amino-4-bis-(β-hydroxyethyl)-aminobenzol als Sulfatsalz) und 0,068 mol/kg Reaktionspartner in Wasser gelöst und
A. entweder mit 25%iger Ammoniak-Lösung auf pH 9,5 eingestellt; oder
B. mit Phosphorsäure auf pH 6,8 eingestellt wurden.

Beide Lösungen wurden jeweils im Verhältnis 1 : 1 mit 6%iger Wasserstoffperoxid-Lösung gemischt und diese Mischung bei Raumtemperatur auf Woll-Läppchen sowie Strähnen aus Büffelhaar, gebleichtem Menschenhaar und normalem braunem Menschenhaar aufgebracht.

Bei den alkalisch eingestellten Präparaten A. betrug die Einwirkungsdauer 15 Minuten, bei den sauer eingestellten Präparaten B. 30 Minuten.

Nach Ausspülen mit Wasser und Trocknen wurden die Ausfärbungen begutachtet. Es wurden folgende Resultate erzielt:

| Zusammensetzung | Reaktionspartner | Farbton |
|---|---|---|
| 1. A (alkalisch) | 4-Amino-2,6-dihydroxypyrimidin | Violett |
| 1. B (sauer) | | Anthrazit |
| 2. A (alkalisch) | 4,5,6-Triaminopyrimidinsulfat | Goldbraun |
| 2. B (sauer) | | Mattblond |
| 3. A (alkalisch) | 2,4-Diamino-6-hydroxypyrimidinhemisulfat | Dunkelblau |
| 3. B (sauer) | | Blau |
| 4. A (alkalisch) | 3-Amino-5,6-dimethyl-1,2,4-triazin | Aschblond |
| 4. B (sauer) | | Mattbraun |
| 5. A (alkalisch) | 2-Amino-4-hydroxy-6-methylpyrimidin | Hellbraun |
| 5. B (sauer) | | Mattblond |
| 6. A (alkalisch) | 4,6-Dihydroxy-2-2-methylpyrimidin | Goldbraun |
| 6. B (sauer) | | Mattblond |
| 7. A (alkalisch) | 2-Amino-4-methylpyrimidin | Goldbraun |
| 7. B (sauer) | | Mattblond |

Die erhaltenen Färbungen erwiesen sich als außergewöhnlich lichtstabil und waren auch nach fünf Haarwäschen noch unbeeinträchtigt.

## Patentansprüche

1. Haarfärbemittel, das als Entwicklersubstanz 1-Amino-4-bis-(β-hydroxyethyl)-aminobenzol und zusätzlich mindestens eine der Komponenten 4,6-Diaminopyrimidin, 4-Amino-2,6-dihydroxypyrimidin, 4,5,6-Triaminopyrimidin, 2,4-Diamino-6-hydroxypyrimidin, 2,4,5-Trihydroxypyrimidin, 2,4,6-Trihydroxypyrimidin, 2-Amino-4-methylpyrimidin, 2-Amino-4-hydroxy-6-methylpyrimidin, 4,6-Dihydroxy-2-methylpyrimidin, 3,4-Diamino-5-hydroxypyrazol und/oder 3-Amino-5,6-dimethyl-1,2,4-triazin bzw. deren wasserlöslichen Salze enthält.

2. Haarfärbemittel nach Anspruch 1, dadurch gekennzeichnet, daß es 1-Amino-4-bis-(β-hydroxyethyl)-aminobenzol in Kombination mit 2,4-Diamino-6-hydroxyprimidin enthält.
